# EUROPEAN PATENT APPLICATION

(11) **EP 3 975 125 A1**
(43) Date of publication of application: **30.03.2022**
(21) Application number: 20197969.7
(22) Date of filing: 24.09.2020
(51) Int. Cl.: G06T 11/00, G06T 7/00

(54) **ANONYMOUS FINGERPRINTING OF MEDICAL IMAGES**

(71) Applicant: Koninklijke Philips N.V., 5656 AG Eindhoven (NL)
(72) Inventor: SOMMER, Karsten, 5656 AE Eindhoven (NL); LENGA, Matthias, 5656 AE Eindhoven (NL); SAALBACH, Axel, 5656 AE Eindhoven (NL)
(74) Representative: Philips Intellectual Property & Standards

(57) **Abstract**

Disclosed herein is a medical system (100, 300) comprising a memory (110, 110') storing machine executable instructions (120, 120') and at least one trained neural network (122). Each of the at least one neural network is configured for receiving a medical image (130) as input. Each of the at least one trained neural network comprises multiple hidden layers. Each of the at least one trained neural network has been modified to provide hidden layer output (124) in response to receiving the medical image. The hidden layer output is outputted directly from one or more of the multiple hidden layers. The medical system further comprises a computational system (104, 104'). Execution of the machine executable instructions causes the computational system to: receive (200) the medical image; receive (202) the hidden layer output in response to inputting the medical image into each of the at least one trained neural network; provide (204) an anonymized image fingerprint (126) comprising the hidden layer output from each of the at least one trained neural network; and receive (210) an image assessment (128) of the medical image in response to querying a historical image database using the anonymized image fingerprint.

## Description

### FIELD OF THE INVENTION

The invention relates to medical imaging such as magnetic resonance imaging, in particular to the difficulty in dealing with errors and abnormal situations when using medical imaging systems.

### BACKGROUND OF THE INVENTION

Various tomographic medical imaging techniques such as Magnetic Resonance Imaging (MRI), Computed Tomography, Positron Emission Tomography, and Single Photon Emission Computed Tomography enable detailed visualization of anatomical structure of a subject. A common problem in applying these imaging modalities is that the interpretation of the medical imaging and the correct operation of the equipment requires years of training. When an operator encounters an abnormal fault or image artifact during image acquisition that the operator not encountered before it may not be possible for the operator to react appropriately.

Gatys et. al, "Image Style Transfer Using Convolutional Neural Networks," Proceedings of the IEEE Conference on Computer Vision and Pattern Recognition (CVPR), 2016, pp. 2414-2423 discloses rendering the semantic content of an image in different styles is a difficult image processing task. Arguably, a major limiting factor for previous approaches has been the lack of image representations that explicitly represent semantic information and, thus, allow to separate image content from style. Image representations derived from Convolutional Neural Networks optimized for object recognition, which make high level image information explicit were used. A Neural Algorithm of Artistic Style that can separate and recombine the image content and style of natural images was also used. The algorithm allows the production of new images of high perceptual quality that combine the content of an arbitrary photograph with the appearance of numerous well-known artworks. Results provide new insights into the deep image representations learned by Convolutional Neural Networks and demonstrate their potential for high level image synthesis and manipulation.

### SUMMARY OF THE INVENTION

The invention provides for a medical system, a computer program, and a method in the independent claims. Embodiments are given in the dependent claims.

Embodiments may provide for an improved means of providing guidance and instructions to the operators of medical imaging systems or an operator or a medical system investigating medical images. One example would be an operator controlling a magnetic resonance imaging system. Another example would be a radiologist at an imaging workstation interpreting medical images.

Embodiments may potentially achieve this by providing a system that creates an anonymized image fingerprint from the medical image and then uses the anonymized image fingerprint to search a historical image database. The historical image database provides an image assessment in response to being queried with the anonymized image fingerprint. The use of the historical image database enables the operator to search for relevant information from previous acquisitions of medical images and need not be limited geographically. When only the medical images from a single imaging site or hospital are considered the amount of information may be limited. If this information is pooled using the anonymized image fingerprint, then data from many sites may be combined while at the same time protecting subject privacy and providing data security.

The anonymized image fingerprint is constructed by inputting the medical image into at least one trained neural network and taking hidden layer output from each neural network. The hidden layer output is also known as a feature vector. This is similar to techniques used in style transfer using a Convolutional Neural Network (CNN), but there are several significant differences. In style transfer only a single neural network may be used. In embodiments more than one neural network may be used. The output of different hidden layers from the same neural network or from multiple neural networks may be combined. For example, they can be concatenated into a larger vector or matrix.

The type and variety of neural networks that can be used to generate the hidden layer output is also very broad. CNNs that have been broadly trained for image classification may be used. Specialized neural networks such as U-Nets for image segmentation of medical images may also be used. As was mentioned above, combinations of these different types of trained neural networks may be used. An advantage is that neural networks that have been trained for other image processing purposes may be used without specialized training.

In the simplest case, the anonymized image fingerprint is constructed from the hidden layer output (feature vectors from the hidden layers). However additional information may also be included such as metadata descripting the type of medical image, the positioning of the subject, the configuration of the medical imaging system when the medical image was acquired. This additional data included could be useful as part of the query for the historical image database as well as for filtering the output of the historical image database.

The anonymized image fingerprint may be sent to the historical image database. The anonymized image fingerprint may be used to search for entries in the database that have a similar image fingerprint. This could be performed using a neural network, but it need not be. Various data clustering techniques or the application of a similarity measure between the anonymized image fingerprint and the image fingerprint of entries in the historical image database may be used to identify entries in the historical image database without training.

The historical image database could for example contain an image fingerprint for each record and then historical data. The historical data could contain all sorts of information such as the configuration of the medical imaging system for a subsequent scan, information about identified anatomical structures, artifacts, or even identified hardware faults. This information can be returned to the operator in a raw form or it may be filtered. An advantage of this historical image database is that the type of information it provides can be adapted on the fly to what the operator needs.

In one aspect the invention provides for a medical system that comprises a memory that stores machine-executable instructions and also stores at least one trained neural network. Each of the at least one neural network is configured for receiving a medical image as input. That is to say each of the at least one neural network is configured to receive image data as input. Each of the at least one trained neural network comprises multiple hidden layers. Each of the at least one trained neural network has been modified to provide hidden layer output in response to receiving the medical image. The hidden layer output is outputted directly from one or more of the multiple hidden layers.

The hidden layer output is taken from the values that neurons produce when the medical image is input into that neural network. The hidden layer output can be provided in different ways. In some examples all of the values of the nodes or neurons within a hidden layer are taken and used as the hidden layer output. In some cases, only a portion of the nodes may be taken. In other examples the values of nodes or neurons from multiple hidden layers are concatenated together to form the hidden layer output. Likewise, the hidden layer output of more than one neural network may be concatenated together. The hidden layer output from a single neural network may be referred to as a feature vector.

The medical system further comprises a computational system. The medical system may take different forms in different examples. In one example the medical system may be a workstation or computer used for processing a medical image such as one would find in a radiology department. In other examples the medical system may be a remote computer system possibly provided by a cloud or internet connection that is used to remotely process medical images. In other examples the medical system may be including the medical imaging system. In this example it can include any variety of imaging modalities such as ultrasound, computed tomography or other nuclear imaging techniques.

Execution of the machine-executable instructions causes the computational system to receive the medical image. Execution of the machine-executable instructions further causes the computational system to receive the hidden layer output in response to inputting the medical image into each of the at least one trained neural network. This for example may involve the computational system being programmed to read the values of the nodes or neurons within each of the at least one trained neural network. Providing the hidden layer output may also involve the concatenation of the output from each of the multiple hidden layers if they are used and/or the multiple trained neural networks.

Execution of the machine-executable instructions further causes the computational system to provide an anonymized image fingerprint comprising the hidden layer output from each of the at least one trained neural network. The hidden layer output may be used to provide an anonymized image fingerprint because they necessarily contain less information than the original medical image. This enables the production of an image fingerprint which is descriptive of the original medical image and its properties but is nonetheless anonymous. This enables it to be provided in a secure way that protects the identify and privacy of the subject for which the medical image was provided.

Execution of the machine-executable instructions further causes the computational system to receive an image assessment of the medical image in response to querying a historical image database using the anonymized image fingerprint. In other words, the anonymized image fingerprint can be sent to either an internal or external database system which then compares it to other image fingerprints which were previously acquired. This may be performed using various search algorithms and artificial intelligence techniques. The image assessment may be data that has been attached to other image fingerprints within this historical image database. This, for example, may be data descriptive of the quality of the image such as image artifacts or the presence of lesions or other non-standard tissue structures as well as the configuration that was used in producing the image or subsequent images. For example, in one instance the image assessment could be the identification of a particular type of image artifact that is present due to motion of the subject or even the failure of a component within a medical imaging system. In other examples the medical image for example could be a scout or survey image that is used in a preliminary fashion. The image assessment for a similar scout image could be the settings that were used to acquire a subsequent diagnostic medical image.

This embodiment has several very significant benefits. Normally it is impossible to maintain large databases of medical image data due to privacy and security concerns. This embodiment provides a way to provide a descriptive anonymized image fingerprint to a large central database. Also, the data to produce the historical image database can be gathered from a variety of sources without the need to worry about security or the disclosure of personal data. The embodiment therefore provides for improved security in medical image databases.

Another significant benefit is that the system does not need to be trained in advance to recognize specific image problems or artifacts or lesions. It also does not need to be trained to recognize various hardware failures. The at least one trained neural network may be constructed from the neural networks that have been trained for processing images in general. For example, an image classification neural network may be used including ones that have not been trained specifically for medical imaging processing such as artifact identification or segmentation. The process of training a neural network for image processing enables it to recognize various combinations and structures.

The at least one trained neural network may also be specifically trained neural networks for performing medical imaging tasks such as image reconstruction or image segmentation. However, the type of neural network which functions is extremely broad. The anonymized image fingerprint then enables the search within the historical image database without prior training. A neural network could naturally be trained and used to match the anonymized image fingerprint with other image fingerprints in the historical image database but this is not necessary. Various metrics and nearest neighbors or clustering techniques which do not need training, can be used to provide the image assessment. This means that the medical system may be enabled to provide an image assessment without specific prior training. This is extremely powerful.

In another embodiment the historical image database is queried via a network connection. For example, in this example the historical image database may be located at a remote or different location.

The use of the anonymized fingerprint over the network connection is extremely beneficial because it provides security and anonymization for the original medical image. This may enable the remote use of the historical image database without an encryption system.

In another embodiment the image assessment comprises an identification of one or more image artifacts. For example, this may be used as an alert to the operator to search or be aware that it is possible the medical image has one or more image artifacts.

In another embodiment the image assessment further comprises an assignment of an image quality value. For example, the images in the historical image database could have been previously assigned an image quality value either manually or via some algorithm. The matching of the anonymized image fingerprint to a nearest or closest neighbor in the historical image database may be used to assign an image quality value. This may for example be a numerical value or it may also be more subjective such as the image being sufficient or insufficient quality that was judged by a radiologist previously.

The image assessment further comprises a retrieved diagnostic guideline. The anonymized image fingerprint may be used to search the historical image database for an image that has similar properties and has a diagnostic guideline attached to it. This may then be provided as the retrieved diagnostic guideline.

The image assessment further comprises instructions to repeat measurement of the medical image. For example, images in the historical image database may have been labeled that the image had failed or did not have a quality or have sufficient noise or artifacts that rendered it not useful for diagnostic use. These could then be detected or found in the process of searching the historical image database with the anonymized image fingerprint. This may be used to provide instructions to repeat measurement of a medical image.

In another embodiment the image assessment comprises a suggested or follow up acquisition and additional medical images. For example, during the search of the historical image database the anonymized image fingerprint may identify images that had structures or lesions which were not readily visible but present in the image. This may then be used to provide data which can be used to generate the suggestion of the follow up acquisitions.

In another embodiment the image assessment comprises an identification of image acquisition problems. This for example may be an error in configuration or setup of the medical imaging system. This may also be indicative of the failure of components of the medical imaging system.

In another embodiment the image assessment comprises identification of an incorrect field of view.

In another embodiment the image assessment further comprises identification of an improper subject positioning.

In another embodiment the image assessment further comprises identification of a regular or inconsistent subject inspiration or breathing.

In another embodiment the image assessment further comprises identification of metal artifacts.

In another embodiment the image assessment further comprises identification of motion artifacts.

In another embodiment the image assessment further comprises identification of foreign objects in the image.

In another embodiment the image assessment further comprises medical image scan planning instructions. For example, the medical image may be a survey or scout scan. This may then be matched with other survey or scout scans to provide the data that can be used for subsequent image acquisition planning. This may be a great aid to the operator of the medical system.

In another embodiment the image assessment further comprises a set of workflow recommendations. These could be retrieved for several or previous images and may be provided to aid the planning of further workflow.

As can be seen above, the image assessment can take a variety of forms. When constructing the historical image database, a variety of types of information can be bundled together in object or file attached to its own image fingerprint for each of its records. In some cases, it may not be desirable to retrieve all of the information that is attached to matching images in the historical image database. In these cases, there may be a filter either within the historical image database or within the medical image itself which restricts or selects what type of information is presented to the operator.

In another embodiment the medical system comprises the historical image database. The historical image database is configured to provide the image assessment by identifying a set of similar images by comparing the anonymized image fingerprint to image fingerprints in the image database. The set of similar images each comprises historical data. The medical system historical image database is further configured to provide at least a portion of historical data as the image assessment. As was mentioned above, this step may involve a filtering step. For example, the operator of the medical system may have a user interface which chooses the type of information which the operator would like to receive. In other examples, the medical system may be preconfigured to filter the historical data. In the case of multiple records matching the anonymized image fingerprint, there may be a selection mechanism that looks for commonality within the historical data.

In another embodiment the comparison between the anonymized image fingerprint to image fingerprints in the image database is performed by applying a similarity measure to the anonymized image fingerprint and each of the image fingerprints.

In another embodiment the comparison between the anonymized image fingerprint to the image fingerprints in the image database is performed by applying a learned similarity measure to the anonymized image fingerprint and each of the image fingerprints.

In another embodiment the comparison between the anonymized image fingerprint to image fingerprints in the image database is performed by applying a metric to the anonymized image fingerprint and each of the image fingerprints. This metric for example, may be a measure of a nearest neighbor.

In another embodiment the comparison between the anonymized image fingerprint to image fingerprints in the image database is performed by calculating a Minkowski distance between the anonymized image fingerprints and each of the image fingerprints.

In another embodiment the comparison between the anonymized image fingerprint to image fingerprints in the image database is performed by calculating an Mahalanobis distance between the anonymized image fingerprint and each of the image fingerprints.

In another embodiment the comparison between the anonymized image fingerprints to image fingerprints in the image database is performed by applying a Cosine similarity measure to a difference between the anonymized image fingerprints and each of the image fingerprints.

In another embodiment the comparison between the anonymized image fingerprints to image fingerprints in the image database is performed by using a trained vector comparison neural network. With the exception of the neural network embodiment described above, the comparison can be performed without training. The use of the trained vector comparison neural network may be beneficial because it for example may be tuned to provide better or more accurate matches. However, this may involve training. For example, a person may have to manually go through the historical image database and retrieve images or records which have relevant data. The training of the trained vector comparison neural network would therefore be very straightforward but involve a significant amount of manual labor.

In another embodiment the neural network is a pre-trained image classification neural network. This embodiment is beneficial because an image classification neural network that has been trained for some other purpose such as recognizing types of animals or other items in the image may be used or redirected for this use.

In another embodiment the neural network is a pre-trained image segmentation neural network. This embodiment may also be beneficial because the pre-trained image segmentation neural network may be re-tasked for embodiments.

In another embodiment the neural network is a U-Net neural network.

In another embodiment the neural network is a ResNet neural network.

In another embodiment the neural network is a DenseNet neural network.

In another embodiment the neural network is an EfficientNet neural network.

In another embodiment the neural network is an Xception neural network.

In another embodiment the neural network is an Inception neural network.

In another embodiment the neural network is a VGG neural network.

In another embodiment the neural network is an auto-encoder neural network that for example may be configured for auto-encoding images.

In another embodiment the neural network is a recurrent neural network.

In another embodiment the neural network is a LSTM neural network.

In another embodiment the neural network is a feed forward neural network.

In another embodiment the neural network is a multi-layer perceptron.

In another embodiment the neural network is a network resulting from a neural network architecture search. For example, any or all of the above neural networks may be available and they may be tested to see which works the best.

In another aspect the provided hidden layer output is provided from any one of the following: a convolutional layer, a dense layer, an activation layer, a pooling layer, an unpooling layer, a normalization layer, a padding layer, a dropout layer, a recurrent layer, a transformer layer, a linear layer, a resampling layer, an embedded representation from an auto-encoder, and combinations thereof. The convolutional layer may for example be a transposed, dilated, or sparse convolutional layer.

The memory further stores a bag-of-words model configured to output a set of image descriptors in response to receiving the medical image. Execution of the machine-executable instructions further comprises receiving the set of image descriptors in response to inputting the medical image into the bag-of-words model. The anonymized image fingerprint further comprises the set of image descriptors.

The medical system further comprises a medical imaging system. Execution of the machine-executable instructions further causes the computational system to control the medical imaging system to acquire medical imaging data. Execution of the machine-executable instructions further causes the computational system to reconstruct the medical image from the medical imaging data.

In another embodiment the medical imaging system is a magnetic resonance imaging system.

In another embodiment the medical imaging system is a computed tomography system.

In another embodiment the medical imaging system is an ultrasonic imaging system.

In another embodiment the medical imaging system is an X-ray system.

In another embodiment the medical imaging system is a fluoroscope, for example a digital fluoroscope.

In another embodiment the medical imaging system is a positron emission tomography system.

In another embodiment the medical imaging system is a single photon emission computed tomography system.

In another embodiment the medical image is a tomographic medical image.

In another embodiment the anonymized image fingerprint further comprises meta data descriptive of a configuration of the tomographic medical imaging system during acquisition of the tomographic medical imaging data. This embodiment may be beneficial because the meta data may be useful in searching for matching image fingerprints.

In another embodiment the image assessment comprises the scan planning instructions. Scan planning may be particularly difficult for untrained operators. The inclusion of scan planning instructions within the image assessment may provide for an automated means of providing scan planning instructions without the need to train the system. Also, the scan planning instructions can be taken from remote locations such as a central database which may even be located in a different country or continent. This enables data and scan planning instructions to be shared in a fashion which provides data security and patient confidentiality. For example, the medical image could be a survey image. The image assessment may then comprise the scan planning instructions which are then provided and used by the operator to assist or configure the medical imaging system to acquire diagnostic medical images.

In another embodiment the medical system further comprises a display. Execution of the machine-executable instructions further causes the processor to render at least the scan planning instructions on the display. This may for example provide a control system for configuring the medical system during the acquisition of further medical images.

In another aspect the invention provides for a method of medical imaging or operating a medical system. The method comprises receiving a medical image. The method further comprises receiving hidden layer output in response to inputting the medical image into each of at least one trained neural network. Each of the at least one trained neural network comprises multiple hidden layers. Each of the at least one trained neural network has been modified to provide hidden layer output in response to receiving the medical image. The hidden layer output is output directly from one or more of the multiple hidden layers. The method further comprises providing an anonymized image fingerprint comprising the hidden layer output from each of the at least one trained neural network. The method further comprises receiving an image assessment of the medical image in response to querying a historical image database using the anonymized image fingerprint.

In another aspect, the invention provides for a computer program comprising machine-executable instructions and at least one trained neural network for execution by a computational system controlling a medical imaging system. Each of the at least one neural network is configured for receiving a medical image as input. Each of the at least one trained neural network comprises multiple hidden layers. Each of the at least one trained neural network has been modified to provide hidden layer output in response to receiving the medical image. The hidden layer output is outputted directly from one or more of the multiple hidden layers. Execution of the machine-executable instructions causes the computational system to receive the medical image.

Execution of the machine-executable instructions further causes the computational system to receive the hidden layer output in response to inputting the medical image into each of the at least one trained neural network. Execution of the machine-executable instructions causes the computational system to provide an anonymized image fingerprint comprising the hidden layer output from each of the at least one trained neural network. Execution of the machine-executable instructions causes the computational system to receive an image assessment of the medical image in response to querying a historical image database using the anonymized image fingerprint.

It is understood that one or more of the aforementioned embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

Medical imaging data is defined herein as being recorded measurements made by a medical imaging system descriptive of a subject. The medical imaging data may be reconstructed into a medical image. A medical image is defined herein as being the reconstructed two- or three-dimensional visualization of anatomic data contained within the medical imaging data. This visualization can be performed using a computer.

K-space data is defined herein as being the recorded measurements of radio frequency signals emitted by atomic spins using the antenna of a Magnetic resonance apparatus during a magnetic resonance imaging scan. Magnetic resonance data is an example of medical image data.

A Magnetic Resonance Imaging (MRI) image or MR image is defined herein as being the reconstructed two- or three-dimensional visualization of anatomic data contained within the magnetic resonance imaging data. This visualization can be performed using a computer. A magnetic resonance image is an example of a medical image.

### BRIEF DESCRIPTION OF THE DRAWINGS

In the following preferred embodiments of the invention will be described, by way of example only, and with reference to the drawings in which:
Fig. 1 illustrates an example of a medical system;
Fig. 2 shows a flow chart which illustrates a method of using the medical system of Fig. 1;
Fig. 3 illustrates a further example of a medical system;
Fig. 4 illustrates the function of a medical system.

### DETAILED DESCRIPTION OF EMBODIMENTS

Like numbered elements in these figs. are either equivalent elements or perform the same function. Elements which have been discussed previously will not necessarily be discussed in later figs. if the function is equivalent.

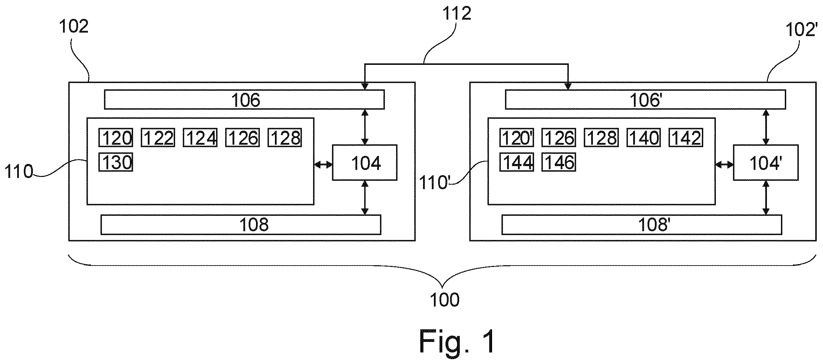

Fig. 1 illustrates an example of a medical system 100. The medical system is shown as comprising a computer 102 with a computational system 104. The computational system 104 could for example represent one or more processing cores at one or more locations. The computational system 104 is shown as being connected to an optional hardware interface 106. The hardware interface 106 may for example be an interface which enables the computational system 104 to communicate with other components of the medical system 100 as well as other computers 102'. For example, the hardware interface 106 could be a network interface. The computational system 104 is further shown as being connected to an optional user interface 108.

The computational system 104 is further shown as being connected to a memory 110. The memory 110 is intended to represent any type of memory which may be accessible to the computational system 104. In this example, the computer 102 is shown as being optionally connected to an additional computer 102'. The additional computer 102' is likewise shown as comprising a computational system 104' that is connected to an optional user interface 108', a hardware interface 106' and a memory 110'. In some examples the medical system 100 only comprises those portions which are part of the computer 102. In other examples, the medical system 100 comprises the components of the computer 102, the optional computer 102' and a network connection 112.

The network connection 112 is shown as connecting the hardware interfaces 106, 106' and enables the two computational systems 104 and 104' to communicate. In yet other examples, the components of the computer 102 and the optional computer 102' are combined with each other. For example, the contents of the memory 110 and the memory 110' may be combined together to form a single computer 102. The computer 102' may for example represent a remote server or cloud-based computer.

The memory 110 is shown as containing machine-executable instructions 120. The machine-executable instructions 120 may for example enable the computational system 104 to provide basic image and data processing tasks as well as control the medical system 100. Likewise, the memory 110' is shown as containing machine-executable instructions 120' that enable the computational system 104' to perform equivalent tasks.

The memory 110 is further shown as containing at least one neural network 122. The at least one neural network 122 have been modified so that they provide hidden layer output 124. The hidden layer output 124 may be considered to be feature vectors which are provided from a hidden layer of the at least one neural network 122. If there is more than one neural network 122 then for example, the hidden layer output 124 from each of the neural networks may be concatenated together. The memory 110 is further shown as containing an anonymized image fingerprint 126. In the simplest case, the anonymized image fingerprint 126 is simply the hidden layer output 124 from the at least one neural network 122. The memory 110 is further shown as containing a medical image 130 that may be input into the at least one neural network 122 to generate the hidden layer output 124.

The anonymized image fingerprint 126 may also contain information that is additional to the hidden layer output 124. For example, there may be meta data associated with the medical image 130 such as the type of scan or other data which may be useful to narrow the search within the historical image database. The anonymized image fingerprint 126 may also for example comprise image descriptors or other data which is descriptive of the medical image 130. The memory 110 is further shown as containing an image assessment 128 of the medical image 130. This is received in response to querying a historical image database 140.

In the memory 110 it is shown that there is the historical image database 140. The historical image database 140 comprises historical images that are associated with image fingerprints. It should be noted that the historical image database 140 could in some examples contain the original images or it may contain just image fingerprints of these historical images. Each of the historical images in the image database or just the image fingerprint is associated with historical data. The historical image database 140 may be queried with the anonymized image fingerprint 126. The computer 102 transferred the anonymized image fingerprint 126 to the computer 102'. The computer 102' then queried the historical image database 140 with the anonymized image fingerprint 126. This resulted in returning a set of similar images 142. The set of similar images 142 comprise historical data 144. The historical data 144 can be considered to be a container for all sorts of information about the images in historical image database 140. For example, if an image had an artifact or it had a failure of a hardware component or there was a particular diagnosis or tumor in one of the historical images, this could be labeled in the historical data 144.

In some cases, the historical data 144 is simply returned as the image assessment 128. In other examples there may also be a filter module 146 which selects the type of data from the historical data 144, which is used to compile or provide the image assessment 128. For example, the filter module 146 may remove certain types of data. For example, if the operator of the medical system 100 is only interested in how to configure the medical system then the filter module 146 may remove other types of data. In other instances, the operator of the medical system 100 may be looking for certain types of tumors or for certain types of image artifacts. This could be selected using the filter module 146 also. The medical system 100 may therefore provide a system which can provide a variety of types of information without prior training. The anonymized image fingerprint 126 may be the individual numerical values from the neurons which were output into the hidden layer output 124. Various grouping and nearest neighbor algorithms may be used to determine the set of similar images 142 without prior training.

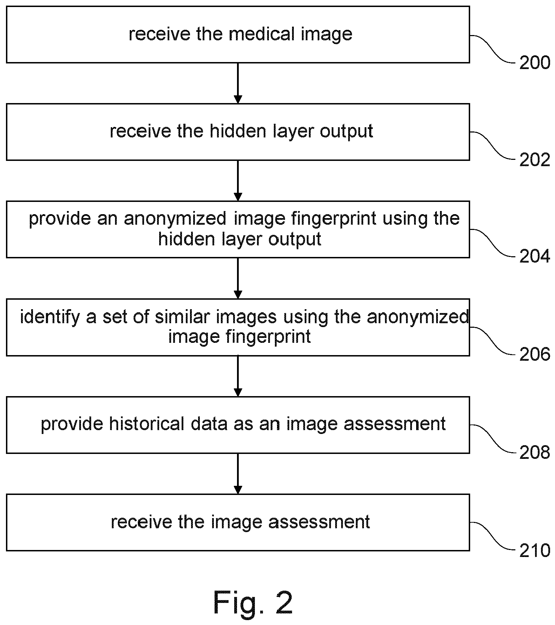

Fig. 2 shows a flowchart which illustrates a method of operating the medical system 100 illustrated in Fig. 1. First, in step 200, the medical image 130 is received. In some examples the medical image 130 may be received by transferring the medical image 130 to the memory 110. In other examples, the medical system 100 may further comprise a medical imaging system and the medical image 130 may be received by acquiring it. Next, in step 202, the hidden layer output 124 is received by inputting the medical image 130 into the at least one neural network 122. Next, in step 204, the computational system 104 constructs an anonymized image fingerprint 126 using the hidden layer output 124. If there is additional meta data or descriptors available, this may in some instances be appended to the hidden layer output 124 to construct the anonymized image fingerprint 126. Next, in step 206, the set of similar images 142 is provided by querying the historical image database 140 with the anonymized image fingerprint 126.

In the simplest case where the anonymized image fingerprint 126 only comprises the hidden layer output 124 there may be some sort of nearest neighbor or metric which is used to compute which historical images belong to the set of similar images 142. In case the anonymized image fingerprint 126 comprises additional descriptors or meta data, these descriptors or meta data may be used to first query the historical image database 140 and reduce the number of images before a metric or nearest neighbor algorithm is used. This for example, may make the system more efficient. Next, in step 208, at least a portion of historical data is provided as the image assessment 128. For example, there may be a filter module 146 which reduces the amount of historical data 144 that is used to construct the image assessment 128. Finally, in step 210, the computer 102 receives the image assessment 128 from the other computer system 102'. As was noted previously, in some examples the computers 102 and 102' may be combined.

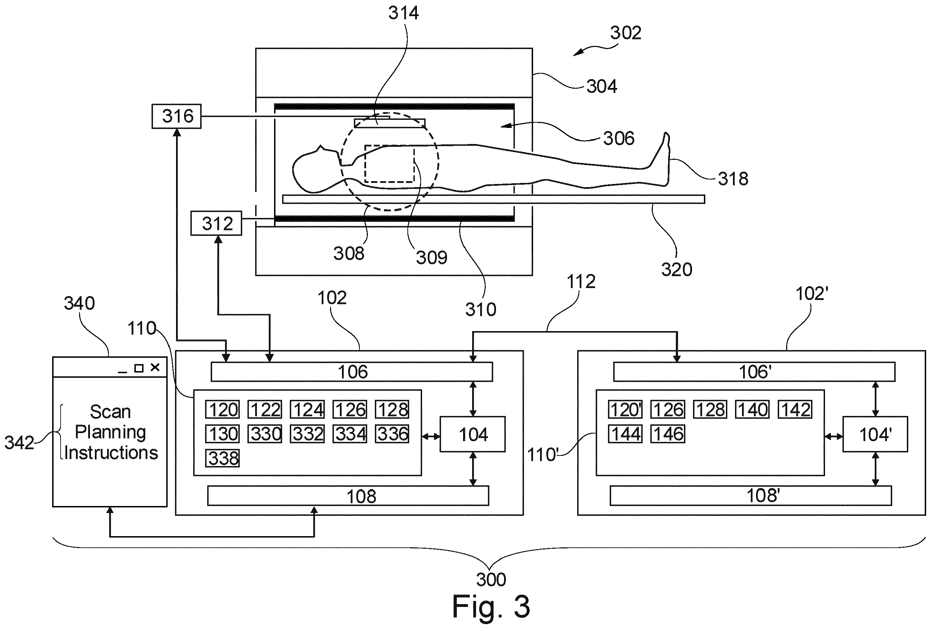

Fig. 3 illustrates a further example of a medical system 300. The medical system 300 is similar to that that is illustrated in Fig. 1 except that it is shown as additionally comprising a magnetic resonance imaging system 302. The magnetic resonance imaging system 302 is intended to depict a medical imaging system in general. For example, the magnetic resonance imaging system 302 may be replaced with a diagnostic ultrasound system, a computed tomography system, a positron emission tomography system or a single photon emission tomography system for example.

The magnetic resonance imaging system 302 comprises a magnet 304. The magnet 304 is a superconducting cylindrical type magnet with a bore 306 through it. The use of different types of magnets is also possible; for instance it is also possible to use both a split cylindrical magnet and a so called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A region of interest 309 is shown within the imaging zone 308. The magnetic resonance data that is acquired typically acquried for the region of interest. A subject 318 is shown as being supported by a subject support 320 such that at least a portion of the subject 318 is within the imaging zone 308 and the region of interest 309.

Within the bore 306 of the magnet there is also a set of magnetic field gradient coils 310 which is used for acquisition of preliminary magnetic resonance data to spatially encode magnetic spins within the imaging zone 308 of the magnet 304. The magnetic field gradient coils 310 connected to a magnetic field gradient coil power supply 312. The magnetic field gradient coils 310 are intended to be representative. Typically magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise the transceiver 316 may also represent a separate transmitter and receivers. The radio-frequency coil 314 may also have multiple receive/transmit elements and the radio frequency transceiver 316 may have multiple receive/transmit channels. For example if a parallel imaging technique such as SENSE is performed, the radio-frequency could 314 will have multiple coil elements.

The transceiver 316 and the gradient controller 312 are shown as being connected to the hardware interface 106 of the computer system 102.

The memory 110 is shown as containing pulse sequence commands 330. The pulse sequence commands are commands or data which can be converted into commands which can be used to control the magnetic resonance imaging system 302 to acquire k-space data 332. The memory 110 is further shown as comprising k-space data 332 that has been acquired by controlling the magnetic resonance imaging system 302 with the pulse sequence commands 330. The computational system 104 may also reconstruct a magnetic resonance image (the medical image 130) from the k-space data 332. The k-space data 332 is intended to represent general medical imaging data.

The memory 110 is further shown as containing meta data 334 which may for example be descriptive of the acquisition of the k-space data 332. For example, it may indicate the type of scan or even the region 309 that was scanned of the subject 318. The meta data 334 may for example be appended to the anonymized image fingerprint 126.

The memory 110 is further shown as containing a bag-of-words model 336. The medical image 130 may for example be input into the bag-of-words model 336 to optionally provide a set of image descriptors 338. Like the meta data 334, if the set of image descriptors 338 are present they may be optionally appended to the anonymized image fingerprint 126. The meta data 334 and/or the set of image descriptors 338 may then be used to optionally query the historical image database 140 to narrow the search for the set of set of similar images 142.

The medical system 300 is shown as further comprising a display 340. The display 340 is shown as depicting scan planning instructions 342 which were generated using the image assessment 128. The scan planning instructions 342 could example be a set of detailed instructions on how to configure the medical system 300 for further medical image acquisition.

As was described above, technologists operating medical imaging systems such as MRI or CT often face unforeseen situations such as image quality problems or unexpected pathologies. Currently, the operator usually has to rely on his/her experience to select the most suitable action, such as modifying the acquisition parameters. This procedure is error-prone, in particular for inexperienced staff members and/or rare events like hardware defects.

Examples may provide for a multi-site workflow assistance tool (medical system) for medical imaging systems is disclosed. It relies on a comparison of the acquired images (medical image 130) with similar cases from multiple other sites (from the historical image database 144), thereby accessing a large body of knowledge. Similarity assessment is based on feature vectors (hidden layer output 124) calculated using a dedicated convolutional neural network (using the at least one neural network 122). Since only these feature vectors (and possibly metadata) are shared between sites, the system does not violate privacy regulations. Alternatively, the system can also be used within single site, e.g. to ensure that internal guidelines for incidental findings are followed.

Advanced imaging systems such as MRI or CT scanners require highly skilled operators. In the clinical routine, technicians must be able to deal with a large variety of unforeseen situations, including image quality problems that require suitable corrective action as well as pathologies that may require insertion of additional dedicated scans. Examples of image quality problems include inappropriate selection of the FOV, incorrect patient positioning, metal artifacts, motion artifacts, or problems due to technical limitations such as unsuccessful preparation phases. In most cases, adequate corrective action will resolve these problems. An example of pathologies that requires additional scans are vascular stenoses, which require angiography and/or perfusion sequences to enable reliable diagnosis.

In the current clinical routine, a technician usually relies on his/her experience to analyze images and remember suitable actions from previous cases. This situation is problematic: a long training time is required to enable new technicians to reliably analyze images and build up enough experience. In addition, many problematic situations such as incidental findings or hardware defects occur very infrequently, so that even experienced technicians may have difficulties to correctly interpret the images. This can be particularly problematic for clinical sites with low case numbers.

In principle, the large amount of medical images that is constantly produced by medical scanners, such as magnetic resonance imaging systems, world-wide could be used to alleviate this problem: comparison with similar images from other sites could often reveal the most suitable action. In particular, smaller sites with a less-specialized healthcare profile may strongly profit from accessing knowledge databases of large sites with specialized departments and staff. Sharing of medical images between clinics is, however, challenging due to strict legal and privacy regulations in many countries.

Examples may provide for a system that enables a fast comparison of medical images with images from multiple other clinical sites (via the historical image database), thereby enabling workflow assistance that relies on a large body of knowledge. The comparison is based on feature vectors (the anonymized image fingerprint 126) that are calculated using a dedicated pre-trained convolutional neural network (CNN), such that only these anonymized vectors need to be shared between sites. Therefore, the system does not violate privacy regulations.

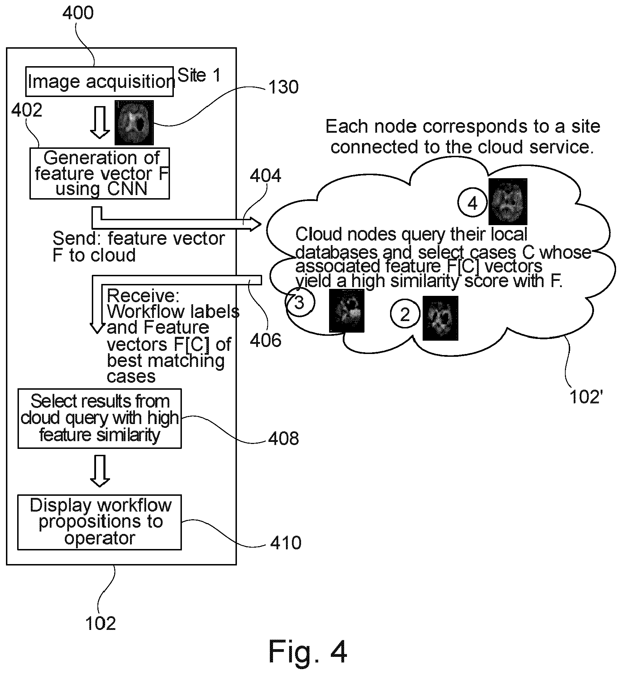

Fig. 4 illustrates a further example of a medical system that may be implemented. Fig. 4 provides a schematic overview of an example, where an unexpected intracranial hemorrhage is depicted. Fig. 4 illustrates this as a flowchart. The steps of the operations are divided into those which would be performed by a computer 102 such as is depicted in Figs. 1 and 3, and a second computer 102' such as is depicted in Figs. 1 and 3 also. Computer 102' is implemented in this example as a cloud-based node.

The method starts in step 400, where an image is acquired. This is the medical image 130. Next, the feature vector is generated 402 using a convolutional neural network. This is equivalent to generating the anonymized image fingerprint 126 using the at least one neural network 122. In this step, the pre-trained CNN is used to calculate a feature vector of the acquired image. In the simplest case, this network is a standard CNN that is trained for image classification, ideally on medical images. In a proof-of-concept study, however, a CNN trained for classification on the ImageNet dataset (i.e. natural images) yielded satisfying results as well. The feature vector is extracted using the output of a hidden layer located deeply within the network (e.g. last convolutional layer before dense layer in ResNet). The extracted feature vector corresponds to a high-level abstraction of the input image data. Image similarity is then assessed by comparing feature vectors of different images with standard metrics such as mean-squared-error (MSE), L1 norm, cosine similarity, etc. or machine-learning based similarity metrics (see Example 3 below).

Importantly, access to the parameters of the CNN should be restricted to ensure that image content cannot be estimated by inverse methods.

Next, in step 404, the feature vector is sent to the cloud-based computer 102'. When the feature vector reaches the cloud-based computer the cloud nodes then query their local databases and select cases or historical images whose associated feature vectors yield a high similarity score with the feature vector f of image 130. In this example, the database is depicted as storing these images. However, the images do not need to be stored in the cloud-based computer 102' itself. The data could be linked to an image fingerprint. This would provide for example a means of anonymizing the historical data of the database contained in the computer system 102'. The calculated feature vector is then sent to a central node, where it is compared to other vectors from different sites. Each of these vectors is stored with a corresponding workflow label that describes the most suitable action, such as adding a specific sequence for further image acquisition. The best matching results are then returned to the querying site (Site 1 in Fig. 4), and the workflow recommendations associated to the best matches (highest similarity score) are displayed to the operator.

Next, in step 406, the workflow labels and feature vectors of the best matching cases are received. In some cases, the feature vectors are not received, only the workflow labels or equivalently the image assessment 128. Next, in step 408, the results are selected from the cloud query with a high feature similarity. This may be performed by the computer 102 or by the computer 102' as was illustrated in Figs. 1 and 3. Finally, in step 410, the workflow or image assessment 128 is displayed to the operator.

For many applications, the workflow labels (used to later provide image assessments 128) can be automatically extracted without requiring manual annotation by the operator:
∘ Insertion of new scans into an imaging protocol can be detected by a simple logfile analysis.
∘ Modification of scan parameters to alleviate artifacts can be extracted by detecting scans that are repeated without modified geometry parameters.
∘ Repositioning of the patient can be detected by analysis of table movement (also usually contained in logfiles).

More detailed annotations can also be obtained by a simple user interaction module that collects feedback for certain situations ("Why was the scan repeated in this way? Was this procedure successful?").

### Examples:

1. In one example, the system is used as an in-house tool within a single clinical site, where the local image archive is used to find the most similar images. This procedure can be desirable if internal guidelines should be followed, such as executing a defined list of additional scans if an incidental finding occurs. In addition, this application scenario has the advantage that details of the previous exams, including images, may be shown to the user with limited privacy concerns.
2. In another example, the medical system is also used for automatic scan planning: After acquisition of a survey/scout scan, the corresponding feature vector is calculated and compared to the vectors from other sites. In this case, the workflow label that is returned to the querying site are the scan planning parameters, i.e. angulation/offset parameters.
3. In another embodiment, the training of the trained neural network is tailored to the targeted image similarity task, e.g. by training the network to produce feature vectors that yield better matches (more similar images). Moreover, instead of comparing the different feature vectors with standard metrics such as MSE, a dedicated network can be trained optimize this comparison (i.e. a machine learning based similarity measure).
   A more comprehensive comparison can also be realized by including system information (e.g., scanner type), protocol information (Tlw, T2w, ...) or even patient information (age, gender, clinical indication...) in the similarity metric.
4. Next to CNN features, also techniques from the computer vision domain could be employed in order to search for similar images. E.g. common approach includes the use of a bag-of-visual-words model. Using such an approach, in a first step, relevant regions in an image are identified using feature detectors, while the regions are characterized in terms of feature descriptors. In a second step, these features are compared to a finite set of codebook entries, the corresponding frequency histogram provides a representation which can be used in a similar fashion as the CNN features for a search for related images.
5. Instead of using a central node that controls the communication with the other sites, a decentralized (p2p) communication between sites can also be implemented.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 102': optional computer
- 104: computational system
- 104': computational system
- 106: hardware interface
- 106': hardware interface
- 108: optional user interface
- 108': optional user interface
- 110: memory
- 110': memory
- 112: network connection
- 120: machine executable instructions
- 120': machine executable instructions
- 122: at least one neural network
- 124: hidden layer output
- 126: anonymized image fingerprint
- 128: image assessment
- 130: medical image
- 140: historical image database
- 142: set of similar images
- 144: historical data
- 146: filter module
- 200: receive the medical image
- 202: receive the hidden layer output in response to inputting the medical image into each of the at least one trained neural network
- 204: provide an anonymized image fingerprint comprising the hidden layer output from each of the at least one trained neural network
- 206: identifying a set of similar images by comparing the anonymized image fingerprint to image fingerprints in the image database
- 208: providing at least a portion of the historical data as the image assessment
- 210: receive an image assessment of the medical image in response to query a historical image database using the anonymized image fingerprint
- 300: medical system
- 302: magnetic resonance imaging system
- 304: magnet
- 306: bore of magnet
- 308: imaging zone
- 309: region of interest
- 310: magnetic field gradient coils
- 312: magnetic field gradient coil power supply
- 314: radio-frequency coil
- 316: transceiver
- 318: subject
- 320: subject support
- 330: pulse sequence commands
- 332: k-space data
- 334: metadata
- 336: bag-of-words model
- 338: set of image descriptors
- 340: display
- 342: scan planning instructions
- 400: image acquisition
- 402: generation of feature vector F using CNN
- 404: send feature vector F to cloud
- 406: receive workflow labels and feature vectors F(C) of best matching cases
- 408: select results from cloud query with high feature similarity
- 410: display workflow propositions to operator

## Claims

1. A medical system (100, 300) comprising:
- a memory (110, 110') storing machine executable instructions (120, 120') and at least one trained neural network (122), wherein each of the at least one neural network is configured for receiving a medical image (130) as input, wherein each of the at least one trained neural network comprises multiple hidden layers, wherein each of the at least one trained neural network has been modified to provide hidden layer output (124) in response to receiving the medical image, wherein the hidden layer output is outputted directly from one or more of the multiple hidden layers;
- a computational system (104, 104'), wherein execution of the machine executable instructions causes the computational system to:
- receive (200) the medical image;
- receive (202) the hidden layer output in response to inputting the medical image into each of the at least one trained neural network;
- provide (204) an anonymized image fingerprint (126) comprising the hidden layer output from each of the at least one trained neural network; and
- receive (210) an image assessment (128) of the medical image in response to querying a historical image database using the anonymized image fingerprint.

2. The medical system of claim 1, wherein the historical image database is queried via a network connection (112).

3. The medical system of claim 1 or 2, wherein the image assessment comprises any one of the following:
- an identification of one or more image artifacts;
- an assignment of an image quality value;
- a retrieved diagnostic guideline;
- instructions to repeat the measurement of the medical image;
- suggestion of follow up acquisition of additional medical images;
- an identification of image acquisition problems;
- an identification of an incorrect field of view;
- an identification of an improper subject positioning;
- an identification of irregular subj ect inspiration;
- an identification of metal artifacts;
- an identification of motion artifacts;
- an identification of foreign objects in the image;
- medical image scan planning instructions;
- a set of workflow recommendations; and
- combinations thereof.

4. The medical system of any one of claims 1, 2, or 3, wherein the medical system comprises the historical image database, wherein the historical image database is configured to provide the image assessment by:
- identifying (206) a set of similar images (142) by comparing the anonymized image fingerprint to image fingerprints in the historical image database, wherein the set of similar images each comprises historical data;
- providing (208) at least a portion of the historical data as the image assessment.

5. The medical system of claim 4, wherein the comparison between the anonymized image fingerprint to image fingerprints in the historical image database is performed using any one of the following:
- applying a similarity measure to the anonymized image fingerprint and each of the image fingerprints;
- applying a learned similarity measure to the anonymized image fingerprint and each of the image fingerprints;
- applying a metric to the anonymized image fingerprint and each of the image fingerprints;
- calculating a Minkowski distance between the anonymized image fingerprint and each of the image fingerprints;
- calculating a Mahalanobis distance between the anonymized image fingerprint and each of the image fingerprints;
- applying a cosine similarity measure to a difference between the anonymized image fingerprint and each of the image fingerprints; and
- using a trained vector comparison neural network.

6. The medical system of any one of the preceding claims, wherein the neural network is any one of the following:
- a pretrained image classification neural network;
- a pretrained image segmentation neural network;
- a U-Net neural network;
- a ResNet neural network;
- a DenseNet neural network;
- an EfficientNet neural network;
- an Xception neural network;
- an Inception neural network;
- a VGG neural network;
- an auto-encoder neural network;
- a recurrent neural network;
- a LSTM neural network;
- a feedforward neural network;
- a multi-layer perceptron; and
- a network resulting from a neural network architecture search.

7. The medical system of any one of the preceding claims, wherein the provided hidden layer output is provided from any one of the following:
- a convolutional layer;
- a dense layer;
- an activation layer;
- a pooling layer;
- an unpooling layer;
- a normalization layer;
- a padding layer;
- a dropout layer;
- a recurrent layer;
- a transformer layer;
- a linear layer;
- a resampling layer;
- an embedded representation from an autoencoder;
- and combinations thereof.

8. The medical system of any one of the preceding claims, wherein the memory further stores a bag-of-words model (336) configured to output a set of image descriptors (338) in response to receiving the medical image, wherein execution of the machine executable instructions further comprises receiving the set of image descriptors in response to inputting the medical image into the bag-of-words model, wherein the anonymized image fingerprint further comprises the set of image descriptors.

9. The medical system of any one of the preceding claims, wherein the medical system further comprises a medical imaging system, wherein execution of the machine executable instructions further causes the computational system to:
- control the medical imaging system to acquire medical image data; and
- reconstruct the medical image from the medical imaging data.

10. The medical system of claim 9, wherein the medical image system is any one of the following: a magnetic resonance imaging system, a computed tomography system, an ultrasonic imaging system, an X-ray system, a fluoroscope, a positron emission tomography system, and a single photon emission computed tomography system.

11. The medical system of claim 10 or 11, wherein the anonymized image fingerprint further comprises metadata descriptive of a configuration of the medical imaging system during acquisition of the medical image data.

12. The medical system of any one of the preceding claims, wherein the image assessment comprises scan planning instructions (342).

13. The medical system of claim 12, wherein the medical system further comprises a display (340), wherein execution of the machine executable instructions further causes the processor to render at least the scan planning instructions on the display.

14. A method of medical imaging, wherein the method comprises:
- receiving (200) a medical image (130);
- receiving (202) hidden layer output (124) in response to inputting the medical image into each of at least one trained neural network (122), wherein each of the at least one trained neural network comprises multiple hidden layers, wherein each of the at least one trained neural network has been modified to provide hidden layer output in response to receiving the medical image, wherein the hidden layer output is outputted directly from one or more of the multiple hidden layers; and
- providing (204) an anonymized image fingerprint (126) comprising the hidden layer output from each of the at least one trained neural network; and
- receiving (210) an image assessment (128) of the medical image in response to query a historical image database using the anonymized image fingerprint.

15. A computer program comprising machine executable instructions (120, 120') and at least one trained neural network (122) for execution by a computational system controlling a medical imaging system (100, 300), wherein each of the at least one neural network is configured for receiving a medical image (130) as input, wherein each of the at least one trained neural network comprises multiple hidden layers, wherein each of the at least one trained neural network has been modified to provide hidden layer output (124) in response to receiving the medical image, wherein the hidden layer output is outputted directly from one or more of the multiple hidden layers, wherein execution of the machine executable instructions causes the computational system to:
- receive (200) the medical image;
- receive (202) the hidden layer output in response to inputting the medical image into each of the at least one trained neural network; and
- provide (204) an anonymized image fingerprint (126) comprising the hidden layer output from each of the at least one trained neural network; and
- receive (210) an image assessment (128) of the medical image in response to querying a historical image database using the anonymized image fingerprint.
